**Europäisches Patentamt**

(19) **European Patent Office**

**Office européen des brevets**

(11) Publication number : **0 143 498 B2**

(12) # NEW EUROPEAN PATENT SPECIFICATION

(45) Date of publication of the new patent specification : **15.05.91 Bulletin 91/20**

(51) Int. Cl.⁵ : **G01N 1/12**

(21) Application number : **84201697.4**

(22) Date of filing : **21.11.84**

(54) **Measurement and/or sampling device for liquid metal and the probe used thereby.**

(30) Priority : **28.11.83 BE 2060271**

(43) Date of publication of application :
**05.06.85 Bulletin 85/23**

(45) Publication of the grant of the patent :
**25.03.87 Bulletin 87/13**

(45) Mention of the opposition decision :
**15.05.91 Bulletin 91/20**

(84) Designated Contracting States :
**DE FR GB IT LU**

(56) References cited :
**CH-A- 409 469**
**DE-C- 1 266 024**
**DE-C- 1 919 117**
**FR-A- 2 389 121**
**FR-A- 2 413 653**

(56) References cited :
**IRON & STEEL INTERNATIONAL, Vol. 51, No. August 1978, pages 223-231, IPC business press, C. LAYCOCK et al.: "Hyprobe-an improved method of sampling liquid steel for hydrogen determination".**
**Prospectus of the firm Ferrotron Elektronik GmbH: "FOX, Ferrotron Oxygen"; Sauerstoff Messsonde für flüssige Metallbäder"; Nr.1 - Stand Januar 1976 with added photos of the sold sampling device (Anlage 2) and a copy of bill no. 395, dated 09.02.76 proving the disposal of 100 sampling devices.**

(73) Proprietor : **ELECTRO-NITE INTERNATIONAL N.V.**
**Amerikalei 110**
**B-2000 Antwerpen (BE)**

(72) Inventor : **Theuwis, Alfons Lambert M.C.**
**Bokrijkseweg 13**
**B-3520 Zonhoven (BE)**
Inventor : **Maes, Joseph Mathy**
**Peerderbaan 114**
**Hechtel (BE)**

(74) Representative : **Debrabandere, René et al**
**BUREAU DE RYCKER Vereenigde Octrooibureaux Belgie N.V. Arenbergstraat 13**
**B-2000 Antwerpen (BE)**

EP 0 143 498 B2

## Description

The invention relates to a converter containing a liquid metal and comprising a sublance as an immersion lance on the end whereof a disposable probe is mounted, said probe comprising a measurement and/or sampling part and a tube carrying said part at its one end, said tube being mounted by its other end against a stop shoulder on the end of the sublance.

By means of the sublance which is situated next to the main lance for oxygen blow, the probe is immersed in the liquid metal bath of the converter.

A converter of this kind is known from DE-C-1 919 117.

The sublance of said device is provided with a narrowed end, over which the tube, mostly made of cardboard, of the disposable probe is pushed under tension against the stop shoulder which is formed by a widening of the lance.

The probe comprises a temperature measuring cell as well as a sample chamber, such as also the sensor described in FR-A-2 389 121 of the applicant, but the probe can comprise either a measuring part or a sample chamber. When assembling the probe on the sublance the measuring part is electrically connected by means of a connector assembled in the tube to contacts assembled on the sublance ; these contacts communicate by means of wires extending throughout the sublance with the measuring instruments proper.

The sublance can have a considerable length which can be 20 m.

Moreover the probe should not only be immersed in relatively calm circumstances in the liquid metal bath but also in turbulent circumstances, namely during the oxygen blow.

By the length of the sublance and especially in beforementioned turbulent circumstance, the sublance is going to vibrate.

Moreover, mainly at the immersion of the probe, liquid metal and slag will splash up.

As a consequence, a burr will be deposited between the stop shoulder of the vibrating sublance and the top end of the tube of the probe.

By the presence of this burr at the end of the sublance, at following use of the sublance the new probe will not join narrowly the stop shoulder of the lance anymore.

This burr, built up at the succesive immersions causes finally that the probe can no more be pushed far enough on the sublance in order to establish a good mechanical connection between the probe and the lance. It is important that, in the case of a probe containing a measuring part, as usual for measurement in a converter, the electrical contact between the connector and the contacts mounted on the lance is no more formed functionally and a correct measurement becomes impossible.

The object of the present invention is to obviate these disadvantages and to provide a measurement and/or sampling device for liquid metal, whereby each deposit of burr between probe and immersion lance is made impossible.

For that purpose the tube, carrying said measuring and/or sampling part at its one end, is mounted by its other end over a part of the sublance on the outside of said sublance, against the stop shoulder on the end of the sublance, and the device further comprises an elastic ring which seals elastically the joining of the tube of the probe against the stop shoulder of the sublance on the outside of said joining, the elastic ring being elastically deformable in that way that, in a state of rest of the tube, the tube of the probe joins the stop shoulder of the sublance. A sampling device for liquid metal comprising a probe wherein elastic rings are used is known from CH-A-409 469, but said rings are used to close hermetically the sampling chamber or the lower end of a chamber with cooling material surrounding the sampling chamber. The probe does not abut against a stop shoulder of the lance and no elastic ring is used to prevent the formation of a burr between the probe and the lance.

FR-A-2 413 653 discloses a sampling and measurement device comprising a probe mounted on the lance by means of a female part fixed on the lance, the probe comprising a tube which is inserted in the female part. The tube does not abut against a shoulder of the lance and the device is not of the kind to which pertains the invention.

No burr can be formed between a shoulder and the probe in said known device, which device does not comprise any elastic ring.

In a particular form of embodiment of the invention the elastic ring is attached to the tube of the probe.

The elastic ring is put on the market together with the probe and also removed together at the remove of the probe after use.

So at every porbe an elastic ring is available, the form of which is adapted to the probe and immersion lance, belonging together.

The invention also relates to a probe which is obviously destinated to be used in a measurement and/or sampling device for use in liquid metal, according to one of the preceding execution variants.

The invention relates especially to such a probe comprising a measurement and/or sampling part and a tube, characterized in that it is provided with an elastic ring at the free end of the tube.

Other details and advantages of the invention will appear by the following description of a measurement and/or sampling device for liquid metal and the probe used therewith, according to the invention. This description is only meant as an example and does not imply a limitation of the invention. The reference numerals concern the annexed drawings.

Figure 1 is a diagrammatical representation of a cross-section of a converter, provided with a measurement and/or sampling device according to the invention during the immersion of the probe.

Figure 2 is a side view of the probe according to the device of figure 1, but drawn on a larger scale.

Figure 3 represents a cross-section representing a probe according to the invention but concerning another form of embodiment of this probe.

Figure 4 represents a cross-section of a probe mounted on the immersion lance, the probe being according to figure 3.

Figure 5 represents a cross-section analogous to those of figure 3 of a probe according to the invention but concerning a further form of embodiment of this probe.

Figure 6 represents a cross-section analogous to those from figure 4 of a probe mounted on an immersion lance but with the probe from figure 5.

Figure 7 represents a cross-section, analogous to the one from figure 6, of the probe mounted on the immersion lance but concerning another form of embodiment of the immersion lance.

Figure 8 represents a cross-section, analogous to those from figures 3 and 5 of a probe according to the invention but relating to still another form of embodiment of this probe.

Figure 9 represents a cross-section, analogous to those of figures 4, 6 and 7 of the probe from figure 8, mounted on the immersion lance.

Figure 10 represents a cross-section, analogous to those from figures 3, 5 and 8, of a probe according to the invention but relating to still another form of embodiment of this probe.

Figure 11 represents a cross-section of the probe from figure 10, mounted on an immersion lance.

Figure 12 represents a cross-section, analogous to those from figures 3, 5, 8 and 10, of a probe according to the invention but concerning still another form of embodiment of this probe.

Figure 13 represents a cross-section of the probe of figure 12, mounted on an immersion lance.

In the different figures the same reference numerals relate to the same items.

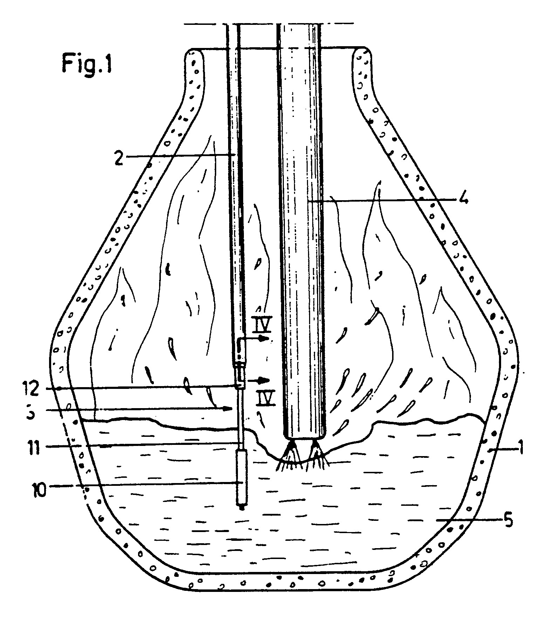

The measurement and sampling device represented in figure 1 is mounted in a converter 1 and comprises in the usual way an immersion lance 2 and a probe 3 mounted thereon.

The immersion lance 2 is the sublance which is inserted vertically, beside the main lance 4 for oxygen blow, in the erected converter, to immerse the probe 3 in the liquid metal bath 5.

As known, this immersion lance 2 is a hollow metal tube, internally provided with a cavity 6 through which extend the electrical wires 7 which connect the probe 3 electrically to the proper measurement equipment.

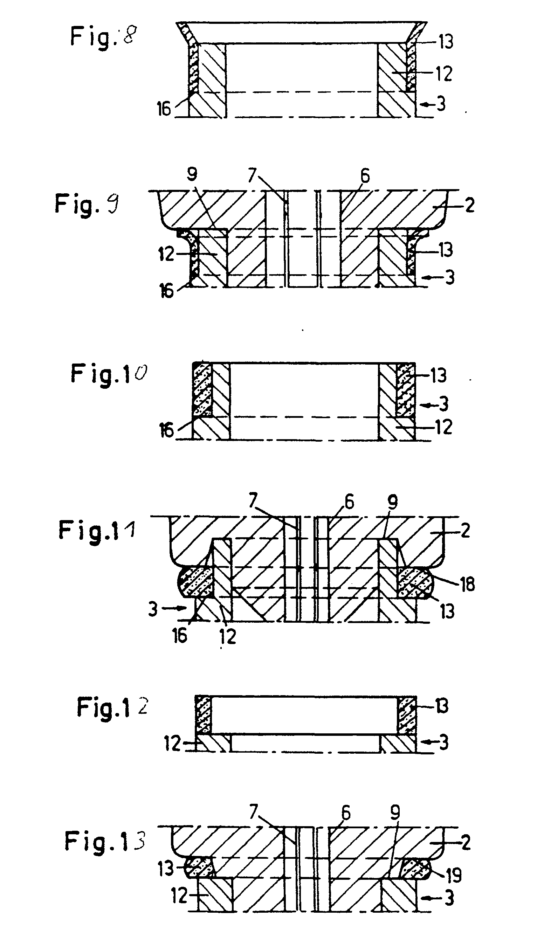

As it appears mainly from figure 4 the immersion lance 2 has downwards a narrowed end 8. The transition from this narrowed end to the broader part forms a stop shoulder 9 for the probe 3.

The probe 3 comprises a measurement and sampling part 10 and a cardboard tube 11, 12 joining thereon, the cardboard tube being provided at its free end with an elastic ring 13.

Except for the elastic ring 13 the construction of the probe 3 is known as such. Therefore, it is not further described in detail. An appropriate probe is among others, described in the French patent nr. 77 13.066 (publication nr. 2.389.121) of the applicant.

The part 10 comprises a sampling chamber and one or more measuring cells, among others for temperature measurement of the liquid metal bath 5. The whole is sidewardly surrounded by a cardboard sleeve 14 and, at the bottom, closed by a metal protection cap 15.

The cardboard tube 11, 12 is for construction reasons, built up by two parts, namely one round part 11 having a diameter smaller than the round sleeve 14 and at the end opposite to the sleeve 14 a shorter round part 12 having the same diameter as the sleeve 14. This diameter is still a little smaller than the diameter of the immersion lance 2.

The inside diameter of the part 12 of the tube is such that the tube 11, 12 fits with some grip over the narrowed end 8 of the immersion lance 2.

When the probe 3 is pushed maximally over the narrowed part 8 of the immersion lance 2 as represented in figure 4, the narrowed end 8 is by its top end relatively deeply inserted in the tube 11, 12. On this top end, contacts are mounted which are connected to the electrical wires 7 and cooperate with a connector mounted inside the tube 11, 12, which connector is electrically connected to the measuring cells of the measurement and sampling part 10. The connector helps to form the mechanical link between the probe 3 and the immersion lance 2.

Characteristic for the invention is the presence of the elastic ring 13 at the free end of the part 12 of the tube 11, 12.

The ring 13 should tighten during the vibrating movement of the probe 3 relatively to the immersion lance 2.

The probe 3 is mounted on the immersion lance 2 in such a way that the ring 13 is partially pushed together, as represented in detail in figure 4.

From this situation the ring must have the possibility to rebound as well as to be compressed further on.

Besides these high demands to the elasticity, the ring 13 should also be sufficiently heat resistant.

A suitable material for the ring 13 that deals with these demands is silicon rubber.

The ring 13 can be a band of elastic material of which the ends preferably overlap each other.

The ring 13 is preferably attached to the end of the tube 11, 12 e.g. by glue or staples, and is put on the market together with the probe 3, destined for one single use.

The ring 13 can, among others depending on the shape of the end of the immersion lance 2 and/or the shape of the end of the tube 11, 12, be of different forms.

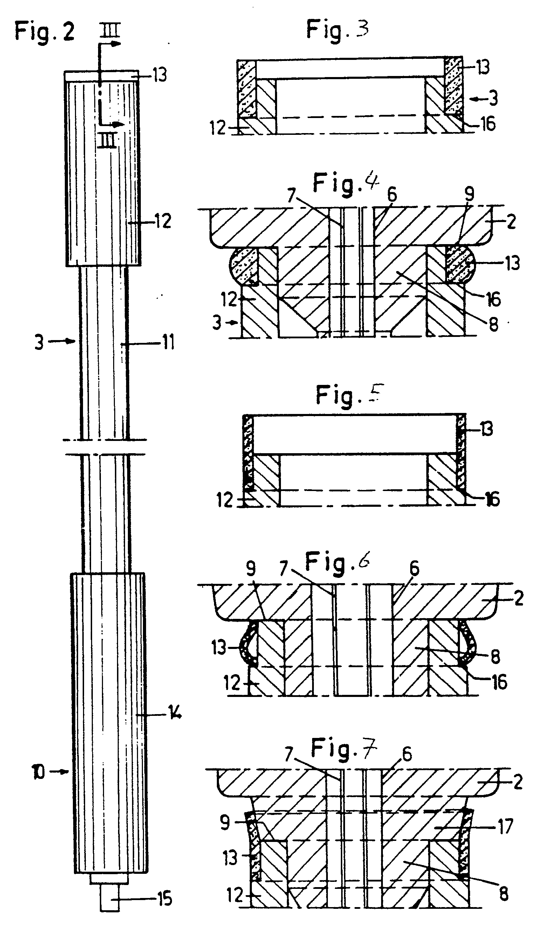

The ring 13 can have a diameter of 1 cm and is flattened by 50% at the mounting of probe 3 to the immersion lance 2. Forms of embodiment of the probe 3 are disclosed corresponding to figures 3 to 13.

In all these forms of embodiment the ring 13 is, when mounted on the immersion lance 2, not completely situated between the extreme edge of the tube 11, 12 and the stop shoulder 9, but it closely joins against the outside of a part of the tube 11, 12 or of the immersion lance 2.

The ring 13 does not obstruct the direct connection of the extreme edge of the part 11, 12 and stop shoulder 9.

The probe to which relate the figures 3 and 4 comprises a closed ring 13 which has not a round but a rectangular section, because its breadth is bigger than its thickness and because the ring surrounds the extreme part of the tube 12 at the outside. Moreover, when the probe is not mounted, the ring protrudes out of the extreme end of tube part 12. Moreover, the extreme end of tube part 12 surrounded by the ring, is narrowed so that the ring 13 is recessed in the outside wall of the tube part 12.

The transition from this narrowed extremity of the tube part 12 to the broader parts forms a stop shoulder 16 for the ring 13.

The ring 13 is attached around the narrowed end of tube part 12 and optionally glued on it.

On the probe, when not mounted, as represented on figure 3, the ring 13 protrudes some what less than half out of tube 11, 12.

The ring 13 has a relatively considerable thickness.

When the probe 3 pushed over the narrowed end 8 up to the stop shoulder 9, the ring 13 is pressed and its outside wall shall bulge as represented on figure 4.

The form of embodiment of the probe according to figures 5 and 6 differs from the form of embodiment according to figures 3 and 4 because the ring 13 is considerably smaller and because exactly half of the width of the ring 13 protrudes out of the tube 11, 12 when the probe is not mounted.

When the tube 11, 12 is slided over end 8 up to the stop shoulder 9 the ring will not swell bulgy but will in whole, bend out bulb-shaped between the stop shoulder 9 and the stop shoulder 16 as is represented on figure 6.

In the device represented in figure 7 the same probe is used as the one represented in figure 5 but the lower end of the immersion lance 2 is, just above the stop shoulder, widened conically up-wards. On the spot of the stop shoulder 9 the diameter of the conical part 17 is equal to the diameter of the narrowed end of the tube 11, 12.

As is clear from figure 7, when the probe is mounted, the elastic ring 13 is not pressed between the stop shoulder 16 of the tube and the stop shoulder 9 of the immersion lance 2 but it is pushed under tension over the conical part 17 whereby it is opened funnel-shaped.

Thus, the ring 13 is mainly strained by stretching and not by pressing so that in this form of embodiment the aforementioned demands of compressibility of the material of the ring 13 are less severe.

Also at the form of embodiment according to figures 8 and 9 the ring 13 is not strained by pressing when the probe 3 is mounted on the immersion lance 2.

The probe 3 is different from the one represented in figure 5 because the part of the ring 13 protruding from the tube part 12 is a little shorter and it widens at the end.

When this probe 3 is mounted on the same immersion lance 2 as represented in figure 6, the ring 13 will not bend out as in the form of embodiment according to figures 5 and 6, but it will bend outwardly at the upper side, against the lower side of the part of the stop shoulder 9 that juts out of the tube 11, 12 as represented in figure 9.

The probe according to figure 10 is fitted with the same ring 13 as the probe according to figure 3, but this ring does not protrude out of the tube 11, 12.

When the probe 3 is mounted on the immersion lance 2 the ring 13 is pressed in the same way as explained with respect to figure 4, not between the stop shoulder 16 and the stop shoulder 9, but between the stop shoulder 16 and a downwards protruding edge 18 that surrounds the stop shoulder 9.

Finally, in the form of embodiment according to the figures 12 and 13, the elastic ring 13 is mounted on the extreme edge of the tube part 12. However, the ring has a rectangular part 12. However, the ring has a rectangular cross-section, having its breadth larger than its thickness and it is situated near the outside.

When the probe 3 is mounted on the immersion lance 2, the ring 13 is not pressed between the tube 11, 12 and the stop shoulder 9, but between the tube and an edge 19 of the immersion lance 2 which is situated higher and which is jutting outwardly.

Between this edge 19 and the stop shoulder 9 the immersion lance is executed conically.

The stop shoulder 9 is only in contact with a part of the extreme edge of the tube part 12.

The invention is not at all restricted to the aforementioned described forms of embodiment and in the framework of the patent application, one can make many alternations to the described forms of embodiment, among others concerning the shape, the composition, the arrangement and the number of the parts used to realize the invention.

Especially, it is not necessary that the elastic ring, in the forms of embodiment wherein it surrounds the cardboard tube, is partially recessed in this tube.

When the ring is recessed, the narrowed end is not necessarily formed by removing material of the tube. In some of these forms of embodiment it is mainly important that, on a distance of the extremity of the tube a stop shoulder for the ring is formed. This stop shoulder can be formed by fitting around the tube on a distance of the extreme edge, a ring having a slightly larger external diameter.

It is not necessary that the tube is a cardboard tube.

It is also unnecessary that the probe comprises both a measuring part and a sampling part. It can also comprise one of these parts but the invention is especially applicable when it comprises a measuring part.

## Claims

1. Converter containing a liquid metal and comprising a sublance (2) as an immersion lance on the end whereof a disposable probe (3) is mounted, said probe (3) comprising a measurement and/or sampling part (10) and a tube (11, 12) carrying said part (10) at its one end, said tube (11, 12) being mounted by its other end over a part of the sublance (2) on the outside of said sublance (2) against a stop shoulder (9) on the end of the sublance (2), and further comprising an elastic ring (13) which seals elastically the joining of the tube (11, 12) of the probe (3) against the stop shoulder (9) of the sublance (2) on the outside of said joining, the elastic ring (13) being elastically deformable in that way that, in a State of rest of the tube, the tube (11, 12) of the probe (3) joins the stop shoulder (9) of the sublance (2).

2. Converter according to the preceding claim, characterized in that the elastic ring (13) is fixed on the tube (11, 12) of the probe (3).

3. Converter according to either one of the claims 1 and 2, characterized in that the elastic ring (13) surrounds a part of the tube (11, 12) of the probe (3).

4. Converter according to the preceding claim, characterized in that the tube (11, 12) of the probe (3) is near its end, provided with an outwardly jutting edge (16) and that the elastic ring (13) is situated at the endside of this edge (16).

5. Converter according to the preceding claim, characterized in that the tube (11, 12) of the probe (3) has a narrowed end and that the edge (16) for the ring (13) is formed by the transition of said narrowed end to a broader part.

6. Converter according to either one of claims 3 to 5, characterised in that the elastic ring (13) which, when the probe (3) is not mounted, protrudes from the end of the tube (11, 12) is pressed directly against the stop shoulder (9) of the sublance (2).

7. Converter according to the claim 4 and 6, characterized in that the elastic ring (13) is pressed between the stop shoulder (9) of the sublance (2) and the recessed edge (16) of the tube (11, 12) of the probe (3).

8. Converter according to claim 6, characterized in that the part of the elastic ring (13) that protrudes at the outside of the tube (11, 12) of the probe (3), which part, when the probe (3) is not mounted, is enlarged at the end, is bent outwardly against a part of the stop shoulder (9) of the sublance (2) that juts out of the tube (11, 12) of the probe (3).

9. Converter according to either one of the claims 3 to 5, characterized in that the elastic ring (13) which, when the probe (3) is not mounted, protrudes at the outside of the tube (11, 12) is elastically stretched around a part (17) of the sublance (2) that enlarges away from the tube (11, 12), said part (17) forming at its bottom side the stop shoulder (9).

10. Converter according to either one of the claims 3 to 5, characterized in that the elastic ring (13) is pressed by an edge (18) of the sublance (2), which edge (18) protrudes downwards and outwards form the stop shoulder (9) and surrounds the latter.

11. Converter according to claim 1, characterized in that the elastic ring (13) is pressed between the end of the tube (11, 12) of the probe (3) and an outwardly jutting edge (19) of the sublance (2) at the outside of a part of the sublance (2) that protrudes out of said edge (19), said part comprising the stop shoulder (9).

12. Converter according to either one of the preceding claims, characterized in that the elastic ring (13) in a free position has a width which is larger than its thickness.

13. Converter according to either one of the preceding claims, characterized in that the elastic ring (13) has such an elasticity that it can meet with a variation of the slit thickness of at least 0.3 cm.

14. Converter according to either one of the preceding claims, characterized in that the elastic ring (13) is made of silicon rubber.

15. Converter according to either one of the preceding claims, characterized in that the elastic ring (13) is elastically deformed against a part of the lance (9, 17, 18, 19).

## Ansprüche

1. Konverter, der ein flüssiges Metall enthält und der eine Sublanze (2) als eine Tauchlanze umfasst an deren Ende eine aufsteckbare Sonde (3) montiert ist, wobei besagte Sonde (3) eine Mess- und/oder Probenahmevorrichtung (10) enthält und ein Rohr (11, 12), an dessen Ende diese Vorrichtung befestigt ist, wobei besagtes Rohr (11, 12) mit seinem anderen Ende über einen Teil der Sublanze (2) an der Aussenseite besagter Sublanze (2) gegen einen Anschlagschulter (9) am Ende der Sublanze (2) montiert ist und weiter einen elastischen Ring (13) enthält, der die Verbindung des Rohres (11, 12) der Sonde (3) gegen den Anschlagschulter (9) der Sublanze (2) an der Aussenseite besagter Verbindung abdichtet wobei der elastische Ring (13) derart elastisch verformbar ist, dass in der Ruhelage des Rohres, das Rohr (11, 12) der Sonde (3) an dem Schulteranschlag (9) der Sublanze (2) anliegt.

2. Konvertor nach dem vorhergehenden Anspruch, dadurch gekennzeichnet, dass der elastische Ring (13) auf dem Rohr (11, 12) der Sonde (3) befestigt ist.

3. Konvertor nach einem der vorhergehenden Ansprüche 1 und 2, dadurch gekennzeichnet, dass der elastische Ring (13) einen Teil des Rohres (11, 12) der Sonde (3) umgibt.

4. Konvertor nach dem vorhergehenden Anspruch dadurch gekennzeichnet, dass das Rohr (11, 12) der Sonde (3) in der Nähe seines Endes mit einer nach aussen hervorstehenden Kante (16) versehen ist und dass der elastische Ring (13) an der Endfläche dieser Kante (16) liegt.

5. Konvertor nach dem vorhergehenden Anspruch dadurch gekennzeichnet, dass das Rohr (11, 12) der Sonde (3) ein verengertes Ende hat und dass die Kante (16) tür den Ring (13) von dem Ubergang des verengerten Endes in einen breiteren Teil gebildet wird.

6. Konvertor nach einem der vorhergehenden Ansprüche 3 bis 5, dadurch gekennzeichnet, dass der elastische Ring (13), der, wenn die Sonde (3) nicht montiert ist, gegenüber dem Ende des Rohres (11, 12) vorsteht, direkt gegen den Anschlagschulter (9) der Sublanze (2) gedrückt wird.

7. Konvertor nach einem der vorhergehenden Ansprüche 4 und 6, dadurch gekennzeichnet, dass der elastische Ring (13) zwischen dem Anschlagschulter (9) der Sublanze (2) und der ausgetieften Kante (16) des Rohres (11, 12) der Sonde (3) eingepresst wird.

8. Konvertor nach Anspruch 6, dadurch gekennzeichnet, dass der Teil des elastischen Ringes (13) der gegenüber der Aussenseite des Rohres (11, 12) der Sonde (3) vorsteht, wenn die Sonde (3) nicht montiert ist, am Ende aufgeweitet ist, nach aussen gegen einen Teil des Schulteranschlages (9) der Sublanze (2) gebogen wird, der gegenüber dem Rohr (11, 12) der Sonde (3) vorsteht.

9. Konvertor nach einem der vorhergehenden Ansprüche 3 bis 5, dadurch gekennzeichnet, dass der elastische Ring (13) der, wenn die Sonde (3) nicht montiert ist, gegenüber der Aussenseite des Rohres (11, 12) vorsteht, elastisch um einen Teil (17) der Sublanze (2) aufgeweitet wird, der sich in der dem Rohr (11, 12) entgegengesetzten Richtung vergrössert, wobei dieser Teil (17) an seiner Bodenseite den Anschlagschulter (9) bildet.

10. Konvertor nach einem der vorhergehenden Ansprüche 3 bis 5, dadurch gekennzeichnet, dass der elastische Ring (13) durch eine Kante (18) der Sublanze (2) zusammengedrückt wird, welche Kante (18) gegenüber dem Anschlagschulter (9) nach unten und aussen vorsteht und diesen umgibt.

11. Konvertor nach Anspruch 1, dadurch gekennzeichnet, dass der elastische Ringe (13) zwischen dem Ende des Rohres (11, 12) der Sonde (3) und einer nach aussen hervorstehenden Kante (19) der Sublanze (2) an der Aussenseite eines Teils der Sublanze (2) zusammengedrückt wird der gegenüber besagter Kante (19) vorsteht, wobei besagter Teil den Anschlagschulter (9) enthält.

12. Konvertor nach einem der vorhergehenden Ansprüche, dadurch gekennzeichnet dass der elastische Ring (13) in einer freien Lage eine Breite hat, die grösser ist als seine Stärke.

13. Konvertor nach einem der vorhergehenden Ansprüche, dadurch gekennzeichnet, dass der elastische Ring (13) eine derartige Elastizität besitzt, dass er eine Änderung der Spaltbreite von wenigstens 0,3 cm überbrücken kann.

14. Konvertor nach einem der vorhergehenden Ansprüche, dadurch gekennzeichnet, dass der elastische Ring (13) aus Silikongummi angefertigt ist.

15. Konvertor nach einem der vorhergehenden Ansprüche, dadurch gekennzeichnet, dass der elastische Ring (13) gegen einen Teil der Lanze (9, 17, 18, 19) elastisch verformt wird.

## Revendications

1. Convertisseur contenant un métal liquide et comprenant une lance secondaire (2), comme lance d'immersion, à l'extrémité de laquelle est montée une sonde (3) à jeter, la sonde (3) comprenant une partie (10) de mesure et/ou de prélèvement d'échantillons, ainsi qu'un tube (11, 12) supportant cette partie (10) à une de ses extrémités, le tube (11, 12) étant monté à son autre extrémité par-dessus une partie de la lance secondaire (2), à l'extérieur de cette dernière, contre un épaulement d'arrêt (9) disposé à l'extrémité de la lance secondaire (2), et comprend, en outre, un anneau élastique (13) qui étanche élastiquement la connexion du tube (11, 12) de la sonde (3) contre l'épaulement d'arrêt (9) de la lance secondaire (2), à l'extérieur de cette connexion, l'anneau élastique (13) étant élastiquement déformable en ce sens qu'à l'état de repos, le tube (11, 12) de la sonde (3) vient s'appuyer contre l'épaulement d'arrêt (9) de la lance secondaire (2).

2. Convertisseur selon la revendication précédente, **caractérisé en ce que** l'anneau élastique (13) est fixé sur le tube (11, 12) de la sonde (3).

3. Convertisseur selon l'une quelconque des revendications 1 et 2, **caractérisé en ce que** l'anneau élastique (13) entoure une partie du tube (11, 12) de la sonde (3).

4. Convertisseur selon la revendication précédente, **caractérisé en ce que** le tube (11, 12) de la sonde (3) est muni, à proximité de son extrémité, d'un bord (16) faisant saillie vers l'extérieur et en ce que l'anneau élastique (13) est situé à l'extrémité de ce bord (16).

5. Convertisseur selon la revendication précédente, **caractérisé en ce que** le tube (11, 12) de la sonde (3) est muni d'une extrémité rétrécie et en ce que le bord (16) destiné à l'anneau (13) est formé par le passage de l'extrémité rétrécie à une partie plus large.

6. Convertisseur selon l'une quelconque des revendications 3 à 5, **caractérisé en ce que** l'anneau élastique (13) qui, lorsque la sonde (3) n'est pas montée, fait saillie depuis l'extrémité du tube (11, 12), est pressé directement contre l'épaulement d'arrêt (9) de la lance secondaire (2).

7. Convertisseur selon les revendications 4 et 6, **caractérisé en ce que** l'anneau élastique (13) est pressé entre l'épaulement d'arrêt (9) de la lance secondaire (2) et le bord évidé (6) du tube (11, 12) de la sonde (3).

8. Convertisseur selon la revendication 6, **caractérisé en ce que** la partie de l'anneau élastique (13) qui fait saillie à l'extérieur du tube (11, 12) de la sonde (3), cette partie, lorsque la sonde (3) n'est pas montée, étant élargie à l'extrémité, fléchit vers l'extérieur contre une partie de l'épaulement d'arrêt (9) de la lance secondaire (2) qui fait saillie hors du tube (11, 12) de la sonde (3).

9. Convertisseur selon l'une quelconque des revendications 3 à 5, **caractérisé en ce que** l'anneau élastique (13) qui, lorsque la sonde (3) n'est pas montée, fait saillie à l'extérieur du tube (11, 12), est élastiquement étiré autour d'une partie (17) de la lance secondaire (2) qui s'élargit à l'écart du tube (11, 12), cette partie (17) formant, sur son côté inférieur, l'épaulement d'arrêt (9).

10. Convertisseur selon l'une quelconque des revendications 3 à 5, **caractérisé en ce que** l'anneau élastique (13) est pressé par un bord (18) de la lance secondaire (2), le bord (18) faisant saillie vers le bas et vers l'extérieur de l'épaulement d'arrêt (9) et entourant ce dernier.

11. Convertisseur selon la revendication 1, **caractérisé en ce que** l'anneau élastique (13) est pressé entre l'extrémité du tube (11, 12) de la sonde (3) et un bord (19) faisant saillie vers l'extérieur de la lance secondaire (2), à l'extérieur d'une partie de la lance secondaire (2) qui fait saillie au-delà du bord (19), cette partie constituant l'épaulement d'arrêt (9).

12. Convertisseur selon l'une quelconque des revendications précédentes, **caractérisé en ce que** l'anneau élastique (13), dans une position libre, a une largeur qui est supérieure à son épaisseur.

13. Convertisseur selon l'une quelconque des revendications précédentes, **caractérisé en ce que** l'anneau élastique (13) a une élasticité telle qu'elle peut faire face à une variation de l'épaisseur de la fente d'au moins 0,3 cm.

14. Convertisseur selon l'une quelconque des revendications précédentes, **caractérisé en ce que** l'anneau élastique (13) est réalisé en caoutchouc silicone.

15. Convertisseur selon l'une quelconque des revendications précédentes, **caractérisé en ce que** l'anneau élastique (13) est élastiquement déformé contre une partie de la lance (9, 17, 18, 19).

Fig.1

Fig. 2

Fig. 3

Fig. 4

Fig. 5

Fig. 6

Fig. 7

Fig. 8

Fig. 9

Fig. 10

Fig. 11

Fig. 12

Fig. 13